# FASCICULE DE BREVET EUROPEEN

(11) **EP 1 361 817 B1**
(45) Date de publication et mention de la délivrance du brevet: **07.09.2005**
(21) Numéro de dépôt: 02706826.1
(22) Date de dépôt: 25.01.2002
(51) Int. Cl.: A61B 1/00

(54) **DISPOSITIF DE REPARATION OSSEUSE**
VORRICHTUNG ZUR KNOCHENVORBEREITUNG
BONE REPAIR DEVICE

(30) Priorité: 25.01.2001 FR 0101011
(43) Date de publication de la demande: 19.11.2003
(73) Titulaire: Cousin Biotech, 59117 Wervicq Sud (FR)
(72) Inventeur: POINTILLART, Vincent, F-33000 Bordeaux (FR); DENEUVILLERS, Guy, F-62155 Merlimont (FR); MELIOT, Sébastien, F-76590 Bertreville Saint Ouen (FR)
(74) Mandataire: Thinat, Michel
(86) Numéro de dépôt international: PCT/FR2002/000311
(87) Numéro de publication internationale: WO 2002/058529

(56) Documents cités:
- EP-A- 0 268 115
- EP-A- 0 475 077
- EP-A- 0 630 625
- EP-A- 0 968 692
- WO-A-01/03614
- WO-A-88/10100
- WO-A-98/26725
- WO-A-99/51171
- DE-U- 9 115 341
- US-A- 5 211 664

## Description

La présente invention a pour objet un dispositif de réparation osseuse par arthrodèse, destiné à la reconstitution d'un os traumatique, notamment pour les vertèbres cervicales.

Dans le cadre des opérations effectuées pour traiter les maladies osseuses de la colonne vertébrale, le chirurgien procède à l'ablation totale ou partielle d'un ou plusieurs corps vertébral. Aucun maintien mécanique n'est plus alors assuré par les os extraits. Pour pallier cet inconvénient il est connu d'utiliser des tiges, généralement en titane et de remplir les espaces laissés vides par les ablations par une matière de remplissage inerte et biocompatible. Une tige est introduite entre deux vertèbres saines pour assurer un premier étayage qui permet d'attendre le durcissement de la matière pâteuse injectée. On peut aussi utiliser un greffon monobloc autologue ou un os de banque ou encore un substitut osseux.

Une autre technique consiste à remplacer la tige et la matière pâteuse biocompatible par une structure grillagée rigide dénommée corps vertébral, ou "vertebral body", dans laquelle est introduit du greffon d'os pour combler le vide laissé par l'ablation et former un support osseux. Mais la mise en place de la cage rigide oblige à distracter les vertèbres adjacentes ce qui entraîne des complications opératoires. La cage est de plus opaque aux rayons X.

Un premier objet de l'invention est de pallier cet inconvénient.

Le document WO-A-98/26 725 divulgue un dispositif de réparation osseuse par greffage comprenant un tube recouvert d'un matériau biorésorbable, dans lequel un greffon peut être introduit, le tube étant constitué à partir d'un treillis métallique.

Selon l'invention, le dispositif de réparation par greffage d'un os traumatisé est caractérisé en ce qu'il comprend un tube de tissu biocompatible à larges mailles, enduit d'un matériau biorésorbable, dans lequel un greffon peut être introduit.
Par tissu, on entendra aussi bien les tricots que les textiles tissés ou non-tissés. Il peut être biorésorbable ou non. Le tube est introduit dans une cavité osseuse en vue de la combler. Mais, de préférence, le tube est maintenu au moyen d'une plaque fixée par vis sur des parties osseuses saines et, dans le cas des vertèbres, le tube est maintenu en position par les plateaux vertébraux des vertèbres supérieure et inférieure.

Le tube est constitué par une enveloppe textile biocompatible, résorbable ou non, à mailles larges. La matière d'enduction peut être un composé de poly-L-Lactide, d'acide polyglycolique ou par leurs copolymères. Le poly-L-Lactide se résorbe dans le temps ce qui permet une croissance du greffon à travers les mailles vers les parties osseuses saines dégagées par l'ablation. Le tube de tissu seul serait trop souple pour assurer un bon contact de l'implant avec les parois osseuses saines de la partie coupée.

Le dépôt de matériau biorésorbable, tel que du PLLA, permet:
- de rigidifier le tube pendant un certain temps;
- de lui donner une forme anatomique adaptée;
- de rendre, dans un premier temps, le tube étanche au passage des greffons afin d'éviter leur dispersion.

Selon une autre caractéristique de l'invention, dans le cas de vertèbres, la plaque sert également à maintenir l'implant en position au moyen d'au moins une vis dont l'extrémité est noyée dans le greffon osseux.

Dans le cas des vertèbres, il est connu de procéder à l'ablation totale ou partielle du corps vertébral par voie antérieure ce qui dégage la place nécessaire pour la mise en place du tube rempli de greffons ou fragments osseux comprimés. La plaque a pour but de maintenir une bonne tenue mécanique de l'ossature et de maintenir le tube incluant un produit de comblement dans une zone précisément délimitée entre les os, en attendant la consolidation osseuse.
Du fait de sa composition, le tube est transparent aux rayons X ce qui permet de voir le développement du greffon osseux par radiographie et de surveiller le développement de la prise de greffe. Le tube de tissu peut être adapté à l'espace intervertébral disponible par simple découpe à l'aide de ciseaux. Lorsque le PLLA se résorbe, les greffons peuvent pousser vers les parois costales et créer une fusion avec ces dernières, ce qui produit l'arthrodèse désirée et le tube devient obsolète.

D'autres caractéristiques et avantages de l'invention apparaîtront au cours de la description qui va suivre d'un mode particulier de réalisation, donné uniquement à titre d'exemple non limitatif, en regard des dessins qui représentent :
- La figure 1, une vue de côté, en coupe verticale partielle de l'implant en position dans la cavité d'une vertèbre ayant subi une corporectomie, entre deux vertèbres saines;
- La figure 2, en vue par-dessus, en coupe horizontale d'une vertèbre ayant subi une corporectomie partielle.

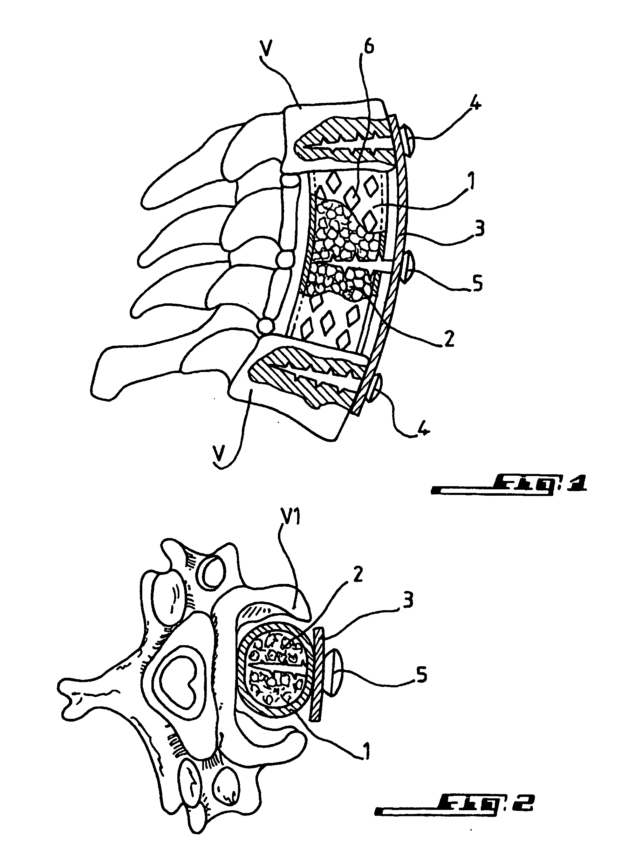

Sur les figures, les mêmes références désignent les mêmes éléments. Sur la figure 1, on voit que l'implant est constitué par un tube 1 sensiblement cylindrique contenant des greffons osseux 2, qui est inclus entre deux vertèbres saines V. Une plaque 3 est appliquée le long du tube 1, la plaque 3 étant vissée dans les vertèbres respectivement supérieure et inférieure à la vertèbre qui a subi une ablation partielle du corps. La plaque 3 est maintenue en position par des vis de maintien 4 à ses deux extrémités, les vis 4 étant fixées dans les deux vertèbres saines V. L'implant est relié à la plaque 3 par au moins une vis 5, qui, dans l'exemple représenté est centrale. Selon l'invention, les mailles larges 6 se dégagent au bout d'un certain temps pour permettre la croissance des greffons. On distingue également sur cette figure des apophyses transverses et épineuses (non référencées) des vertèbres.
La figure 2 représente une vue par-dessus de l'implant en position à l'intérieur d'un corps de vertèbre V1 découpé. On a schématisé, sur cette figure, le canal médullaire (non référencé). Au centre de la plaque 3, on distingue la vis 5 qui pénétrant à travers le tube 1 se fixe dans la masse de greffons osseux pour donner une sécurité dans le maintien du tube 1.

Bien que la description qui précède concerne les vertèbres, l'invention peut être appliquée dans tous les cas où il s'agit de combler des cavités entre deux os, notamment en cas d'ablation.

Il va de soi que de nombreuses variantes peuvent être apportées, notamment par substitution de moyens techniques équivalents, sans sortir pour cela du cadre de l'invention.

## Revendications

1. Dispositif de réparation osseuse par greffage et arthrodèse d'un os traumatisé **caractérisé en ce qu'**il comprend un tube (1) de tissu à larges mailles biocompatible, biorésorbable ou non, enduit d'un matériau biorésorbable, dans lequel un greffon (2) peut être introduit.

2. Dispositif de réparation selon la revendication 1,
**caractérisé en ce qu'**il comprend une plaque (3) de maintien en position du tube (1), la plaque (3) étant capable d'être fixée par des vis (4) sur des parties osseuses saines (V).

3. Dispositif de réparation selon la revendication 2,
**caractérisé en ce qu'**il comprend une vis (5) de fixation du tube (1) sur la plaque (3), dont l'extrémité est capable d'être noyée dans le greffon osseux (2).

4. Dispositif de réparation selon l'une des revendications 1 à 3, **caractérisé en ce que** le greffon osseux (2) est constitué par un ensemble de fragments osseux comprimés et/ou de substituts osseux.

5. Dispositif de réparation selon l'une quelconque des revendications précédentes, **caractérisé en ce que** le matériau biorésorbable d'enduction est à base de poly-L-Lactide, et/ou d'acide polyglycolique et ou de leurs copolymères.

6. Dispositif de réparation selon l'une quelconque des revendications précédentes **caractérisé en ce que** le tissu constituant le tube (1) est un textile tissé ou non tissé, ou un tricot.

7. Dispositif de réparation selon l'une quelconque des revendications précédentes, **caractérisé en ce qu'**il est destiné à être disposé dans une cavité résultant de l'ablation d'une partie de l'os.

## Patentansprüche

1. Vorrichtung zur Knochenreparatur durch Transplantation und Arthrodese eines verletzten Knochens, **dadurch gekennzeichnet, dass** diese einen breitmaschigen, biokompatiblen, biologisch resorbierbaren oder nicht Gewebeschlauch (1) umfasst, der mit einem biologisch resorbierbarem Material beschichtet ist, in den ein Transplantat (2) eingeführt werden kann.

2. Reparaturvorrichtung nach Anspruch 1, **dadurch gekennzeichnet, dass** diese eine Platte (3) aufweist, um den Schlauch (1) in Position zu halten, wobei die Platte (3) mit Schrauben (4) auf den gesunden Knochenteilen (V) befestigt werden kann.

3. Reparaturvorrichtung nach Anspruch 2, **dadurch gekennzeichnet, dass** diese eine Schraube (5) zur Befestigung des Schlauchs (1) auf der Platte (3) aufweist, deren Ende in das Knochentransplantat (2) versenkt werden kann.

4. Reparaturvorrichtung nach einem der Ansprüche 1 bis 3, **dadurch gekennzeichnet, dass** das Knochentransplantat (2) von einer Anhäufung komprimierter Knochenfragmente und/oder von Knochenersatzmitteln gebildet wird.

5. Reparaturvorrichtung nach einem der vorhergehenden Ansprüche, **dadurch gekennzeichnet, dass** das biologisch resorbierbare Beschichtungsmaterial auf Poly-L-lactid und/oder Polyglycolsäure und/oder ihren Copolymeren basiert.

6. Reparaturvorrichtung nach einem der vorhergehenden Ansprüche, **dadurch gekennzeichnet, dass** das den Schlauch (1) bildende Gewebe ein gewebtes Textil oder Vlies oder ein Gewirk ist.

7. Reparaturvorrichtung nach einem der vorhergehenden Ansprüche, **dadurch gekennzeichnet, dass** diese dazu vorgesehen ist, in einer Kavität angeordnet zu werden, die Ergebnis der Abtragung eines Teils des Knochens ist.

## Claims

1. A bone repair device by grafting and arthrodesis of a traumatized bone, **characterized in that** it comprises a biocompatible wide mesh tissue tube (1), either bioresorbable or not, coated with a bioresorbable material, into which a graft (2) may be introduced.

2. The repair device according to claim 1,
**characterized in that** it comprises a plate (3) for maintaining the tube (1) in position, the plate (3) being able to be secured by screws (4) on healthy bone portions (V).

3. The repair device according to claim 2,
**characterized in that** it comprises a screw (5) for securing the tube (1) on the plate (3), the end of which is able to be embedded into the bone graft (2).

4. The repair device according to any of claims 1 to 3, **characterized in that** the bone graft (2) is formed by a set of compressed bone fragments and/or bone substitutes.

5. The repair device according to any of the preceding claims, **characterized in that** the coating bioresorbable material is based on poly-L-lactide, and/or polyglycolic acid and/or their copolymers.

6. The repair device according to any of the preceding claims, **characterized in that** tissue forming the tube (1) is a woven or non-woven fabric, or a knit.

7. The repair device according to any of the preceding claims, **characterized in that** it is intended to be positioned in a cavity resulting from ablation of a portion of the bone.
